# EUROPEAN PATENT SPECIFICATION

(11) **EP 0 422 118 B1**
(45) Date of publication and mention of the grant of the patent: **12.01.1994**
(21) Application number: 89908236.6
(22) Date of filing: 29.06.1989
(51) Int. Cl.: A61L 25/00, A61L 15/16, A61L 31/00

(54) **DRESSINGS**
WUNDVERBAND
PANSEMENTS

(30) Priority: 29.06.1988 GB 8815435
(43) Date of publication of application: 17.04.1991
(73) Proprietor: SMITH & NEPHEW P.L.C., London WC2R 3BP (GB)
(72) Inventor: LLOYD, Ronald, Herts CM21 9NE (GB)
(74) Representative: Gilholm, Stephen Philip
(86) International application number: GB8900725
(87) International publication number: WO9000066

(56) References cited:
- EP-A- 0 091 800
- EP-A- 0 114 581
- WO-A-88/01878
- WO-A-89/01346
- FR-A- 2 542 201
- GB-A- 2 093 190

## Description

This invention relates to systems for forming dressings on the skin, to dressings formed therefrom and to methods of forming such dressings.

Filmic products employed in health care today include wound dressings, surgical incise drapes and I.V. dressings which are used, for example to cover and secure cannulae in place at intravenous access sites.

Known filmic products generally comprise a filmic backing layer in which there is a layer of pressure sensitive adhesive upon substantially the whole of one surface thereof. The adhesive layer may be a discontinuous coating or a continuous coating of, preferably a water-permeable adhesive. Water-absorbent adhesives are preferred because of their moisture vapour permeability.

Moisture vapour permable incise drapes have been disclosed in British Patent No. 1280631 and in United States Patent No. 3645834, European Patent Applications Nos. 51935, 178740, 196459 and United States Patents Nos. 4372303 and 4374520. The known incise drapes of this type in commercial use have proved useful because they held prevent ingress of bacteria into the wound and some reduce bacterial contamination of the surrounding skin.

Certain known incise drapes have the added advantage that they do not cause maceration of healthy skin to which they may be applied because both the film and ahdesive layer are moisture vapour permeable and generally provide the drape with a moisture vapour transmission rate (MVTR) of about between 300 and 800 g/m²/24hr at 37°C and 100% to 10% relative humidity difference when the adhesive is in contact with moisture vapour.

A disadvantage which may arise with known incise drapes is that if the patient sweats profusely the adhesive may be affected and the drape may lift away from the skin and thereby may compromise the sterility of the operation site.

It has been suggested that high moisture vapour permeabilities may be obtained by employing a discontinuous layer of adhesive (see for example European Patent No. 91800). However this is not appropriate for incise drapes where gaps in the adhesive are not acceptable.

It would be advantageous therefore if a drape could be found possessing a sufficiently high moisture vapour transmission rate and water absorption capacity so that it could be adhered to the skin as a substantially continuous unitary layer whereby the disadvantages of both continuous and discontinuous adhesive layers could be avoided. It would be a further advantage if there was little or no propensity for irritation to be caused even after prolonged contact of the drape with the skin.

A further problem common to adhesive dressings used for incise drapes as well as other applications is that of selfadhesion. This problem can be particularly severe when handling large size dressings.

If parts of the adhesive surface are allowed to touch each other those parts will adhere to each other (block) causing rucks or folds in the dressing. The presence of such folds, besides being unsightly, may introduce pathways by which bacteria can enter the wound area or by which exudate can uncontrollably escape. Pulling the self-adhered parts apart is likely to destroy the adhesive layer at that point promoting the disadvantage associated with discontinuous adhesive layers.

Additional problems may also occur with elastomeric films in that pulling of adhered parts may cause the film to permanently stretch. The distorted film may no longer be capable of being laid down flat, again following tracts for bacterial infection.

We have now found that the disadvantage of the prior art can be significantly reduced or eliminated by providing a system for forming a dressing on the skin which comprises a film which is not adhesive per se but which can be applied to the skin as a continuous layer.

Therefore according to the present invention there is provided a two component system for forming a dressing on the skin which comprises a first component comprising a solution of a film forming non-tacky skin adhering hydrophilic polyether polyurethane comprising ether blocks derived from polyethylene glycol and polypropylene glycol and further comprising residues of chain extenders such as aliphatic diols or aromatic diamines, and a second component comprising a non skin-adhering film of a second polymer which will autohesively bond to a film of first polymer,
characterised in that the chain extenders and glycols are present in a mole ratio of diol to glycol in the range of from 2:1 to 1:1. The solvents employed for the solution of first polymer should be pharmacologically acceptable for topical application.

In the system of the invention the films of the second component will autohesively bond to the film of skin adhering polymer formed from the first component that is the two films adhere by autohesion. Autohesion as used herein means the ability of two contiguous polymer surfaces which have substantially no adhesive properties, to bond together. Such a bond is believed to be achieved by diffusion of polymer molecules between the contiguous surfaces of the two polymer films. Normally, the two polymers will, generally, be similar although they need not be chemically identical.

The film components used or formed in the system are substantially non-adhesive per se and thus are non-skin adhering or tacky.

The formed filmic compositions are suitable for use in situ as dressings such as those employed for surgical incise drapes, I.V. dressings and wound dressings or the like. Because such films comprising the second component are not adhesive per se they can be employed as large sheets and can be readily handled.

Although such films are readily handled and can be laid down and smoothed to remove wrinkles, they are not adherent per se to the skin. They can be rendered adherent by contact with the film formed from the polymer present in the first component.

The dressing of the present invention may be formed according to a method which comprises applying to an area of skin a first component comprising a solution of film forming first polymer, in a solvent which is pharmacologically acceptable for topical application, allowing the solvent to evaporate to form a skin adhering film layer and applying over the thus formed film a second component comprising a film of a second polymer. Preferably the second polymer is insoluble in the first component. The film of the second polymer will autohesively bond with the skin adherent film layer of first polymer thereby to form on the skin a dressing which is preferably both bacteria proof and moisture vapour permeable.

In application, using the two-component system, a liquid component comprising the first polymer in solution is first applied to the skin around or over the area to be covered and thereafter, eg. when solvent has dried a sheet or strip of the filmic backing component is applied to the skin over the area to which the liquid has been previously applied. The second polymer thus adheres to the polymer constituting the backing layer.

Thus a dressing can be formed in situ on to the skin which is suitable for use for example as a surgical incise drape.

Such a dressing will comprise a non-tacky skin-adhering filmic first polymer autohesively bonded to second component which comprises a non skin adherent film of a second polymer. Such a dressing is preferably both moisture vapour permeable and bacteria proof.

The liquid first component of the dressings of the invention may be liquid per se for direct topical application by, for example, swab or brush or may be absorbed onto a fabric for use as a wipe. In another embodiment the liquid may be applied as spray either from a pump action dispenser or as an aerosol together with a suitable propellant. Upon drying the polymer will adhere to the skin. However, when dry the formed film has no pressure sensitive adhesive properties.

The dressing materials of the present invention as would be formed in situ on the skin may exhibit different moisture vapour transmission rates depending upon the materials employed and whether the skin contacting layer of the dressing is in contact with moisture vapour or with water.

The moisture vapour transmission rates of the dressing of the present invention may be determined by first applying the first component to a releasable substrate such as siliconised paper and, when dry overlaying the second component. The 'laminate' is then released from the substrate.

The moisture vapour transmission rate may be measured by a procedure known as the Payne Cup method. The method uses a cup 1.5cm deep with a flanged top. The inner diameter of the flange is such as to provide an area for moisture vapour transmission of 10cm² . In this method 10ml of distilled water is added to the cup and a sample of the laminate under test, large enough to completely cover the flange, is clamped over the cup. The complete assembly is then weighed and placed in a cabinet where the temperature and relative humidity are maintained at 37°C and 10% respectively. After 17 hours the cup is removed from the cabinet and allowed to cool at room temperature. After re-weighing, the mass of water lost by vapour transmission is calculated and the result expressed as in g/m²/24hrs at 37°C at 100% to 10% relative humdity difference. Hereinafter the units for moisture vapour transmission will be abbreviated to g m⁻².

The cup is maintained in an upright position during measurement of the MVTR of a sample in contact with moisture vapour and in an inverted position during measurement of the MVTR of a sample in contact with water.

The moisture vapour transmission rate when in contact with moisture vapour but not water is referred to herein after as the upright moisture vapour transmission rate (upright MVTR). Suitably the dressing materials of the present invention may have an upright MVTR of at least 300g m⁻², suitably at least 500g m⁻², greater than 800g m⁻². More suitably the upright MVTR will be greater than 1000g m⁻², most suitably more than 1200g m⁻² and preferably will have an upright MVTR of greater than 1400g m⁻² under the conditions specified above.

The moisture vapour transmission rate when in contact with water is referred to hereinafter as the inverted moisture vapour transmission rate (inverted MVTR) and may be measured by the method described hereinafter. Suitably the dressing materials of the present invention will have an inverted MVTR of greater than 2400g m⁻² humidity difference. More suitably, depending upon the particular application the inverted MVTR may be greater than 4000g m⁻², most suitably it is greater than 6000g m⁻² and preferably greater than 10,000g m⁻².

Preferably the polymers constituting the formed filmic composition will be such as to permit visual observation of the skin, or in the case of an I.V. dressing, of the cannula site through the dressing when it is in place. The composition should preferably be translucent and more preferably transparent.

When the dressings of the invention are employed as surgical incise drapes they are applied to an operation site prior to incision through the skin. Usually the drape remains in position during the operation and is removed afterward. In some cases at the end of the operation the drape may be left in position and a second drape or dressing applied over the first drape. In this instance it is particularly desirable that the first drape should have a large upright and inverted moisture vapour transmission rate to avoid causing maceration to the underlying healthy skin.

An incise drape is adhered to intact skin and it is found that in certain patients sweat may collect beneath known incise drapes and effect their adherency. To avoid moisture gathering beneath the drape, it is now thought that the drape should have a moisture vapour transmission rate of greater than 500g m⁻² 24h⁻¹ at 37°C and 100% to 10% relative humidity difference when in contact with moisture vapour and greater than 2500g m⁻² 24h⁻¹ when in contact with water.

The polymeric material forming the second component may be one used to form any moisture vapour permeable material suitable for use in incise drapes, I.V. dressings and wound dressings. Suitable materials include polyurethanes such as polyester polyurethanes or polyether polyurethanes, polether polyester, polyether polyamides, polymer blends of incompatible polymers, cellulosics polyvinyl acetate derivatives or the like. Other materials which may be employed as the insoluble polymer include rubbers marketed under the trade mark Cariflex, polyisobutadiene, ethyl vinyl acetate polymers and copolymers or blends thereof, eg. with high impact polystyrene, and polyacrylates and methacrylates.

Preferred polyurethanes for use as the second component are thermoplastic polyether polyurethanes. Other hydrophilic polyurethanes which are polyester polyurethanes or which have potentially reactive substituents such as hydroxyl or carboxyl groups are less preferred. Examples of suitable thermoplastic polyurethanes are the linear polyester and polyether polyurethanes known as Estanes (trade mark).

Apt polyether polyurethanes for use in this invention will be random polymers containing units derived from a dihydroxy compound and di-isocyanates.

Aptly the ether units in the hydrophilic polyurethane for use in this invention will be notionally derivable from ethylene diol and propylene or butylene diol; that is they will contain CH₂ CH₂ O-units and -CH₂CH₂CH₂O- and -CH₂CH(CH₃)O- or -(CH₂)₄O-mixtures thereof of which poly -CH₂CH(CH₃)O- blocks are preferred. Desirably the mole ratio of poly(ethylene glycol) to poly[(prop or but)ylene glycol] derivable blocks present in the hydrophilic polyurethanes vary between 1:1 to 1:30, more suitably from 1:2 to 1:10 and preferably from 1:2.5 to 1:4. The molecular weights of these blocks is aptly from 600 to 60000 and favourably from 900 to 4000, for example 1000 to 2000.

Less preferred are hydrophilic polyurethanes which contain poly(ethylene-glycol) derived blocks alone together with a higher proportion of chain extender and di-isocyanate.

Normally and preferably the hydrophilic polyurethane used in the devices of this invention is essentially a single type of polymer (a product of the polymerisation of the same materials) although the blends may be employed to form the hydrophilic polyurethane if desired.

Preferably, the hydrophilic polyurethane for use in this invention will contain residues of aliphatic diols of up to 10 carbon atoms and more suitably up to 4 carbon atoms as chain extenders. The mole ratio of diol to polyglycol used in the preparation of the polymer is within the range of 1:1 to 2:1. A preferred diol is ethane diol in a molar ratio from 1:1 to 2:1. Mole ratios of less than 1 mole ethane diol per mole of polyglycol increases the tendency for a permanent residual tack to be inputed to the first component even when it has dried, whereas ratios of greater than 2 to 1 increases the hard sector content of the polymer which may cause adhesive/cohesive failure between the first and second components, when applied.

Equivalent quantities of aliphatic diamine or aliphatic amine chain extenders, eg ethylene diamine may also be used. Similarly somewhat less aptly than using aliphatic diol chain extenders, the hydrophilic polyurethane may employ an aromatic diamine such as phenylenediamine, benzidine or diaminodiphenylmethane.

The hydrophilic polyurethane will contain sufficient di-isocyanate residues to produce the water contents set forth hereinbefore when the polymer is hydrated.

Most aptly the hydrophilic polyurethane for use in this invention will contain di-isocyanate residues which may be residues of aromatic or aliphatic di-isocyanates such as 4,4'-diphenylmethane di-isocyanate, toluene di-isocyanate, 1,6-hexamethylene di-isocyanate, 4,4'-dicyclohexylmethane di-isocyanate or the like. Favoured di-isocyanates for use in the hydrophilic polyurethane of this invention are 4,4'-dicyclohexylmethane di-isocyanate (which is preferred) and 4,4'-diphenylmethyl di-isocyanate.

The material which forms the second component or backing layer should itself have a moisture vapour transmission rate of above 300g m⁻² 24h⁻¹ at 37°C and 100% to 10% relative humidity difference, more suitably more than 800g m⁻² 24h⁻¹ typically from 1200 to 2000g m⁻² 24h⁻¹ when in contact with moisture vapour.

The material which forms the backing layer should preferably provide an in situ dressing or drape with an inverted moisture vapour transmission rate of greater than 2500g m⁻² 24h⁻1 at 37°C, more suitably, for wound dressings in the range of from 3000 to 18000g m⁻² 24h⁻1 and preferably from 5000 to 12000g m⁻² 24h⁻1 .

Suitably the backing layer will have a thickness of from 10 to 80µm, more suitably 20 to 50µm and preferably from 25 to 40µm.

The soluble polymer present in the first component may be a similar material to that employed for the second polymer and can be a polyacrylate, a polyester, a polyamide or a polyurethane or blends. A particularly preferred polymer is one which is capable of containing from 5 to 95% by weight of water when hydrated.

A preferred polymer, is a hydrophilic polyurethane. Suitable hydrophilic polyurethanes include those having the composition and prepared by the process described in British Patent No. 2093190B. The most suitable hydrophilic polyurethanes are those which will contain up to 95%, preferably from 5 to 50% by weight of water when hydrated and especially those which contain from 10 to 40% by weight of water.

It has been observed that the adhesion between a primer layer of the first component and the filmic second component will be effected by the thickness of the primer layer. Thus in order to maintain integrity of the two components it is preferred that the first component be formulated such that a primer layer of a thickness of from 10µm to 80µm.

Thickness of the primer layer also effects the adhesion between the primer layer (first component) and its substrate ie. skin. It is therefore more preferred that the thickness of the primer layer be about 15µm.

It is further preferred that the soluble and insoluble polymers forming, respectively the first and second components are both of the same class of polymers. A preferred pair comprises two polyurethanes formulated such that one is soluble in a solvent, for example an organic solvent such as isopropanol whilst the other is not. Polyurethanes containing long chain aliphatic isocyanates tend to be soluble in isopropanol whereas those formulated using short chain aliphatic isocyanates, eg. not more than four carbon atoms or aromatic isocyanates tend not to be soluble in isopropanol.

The liquid component containing the first polymer should be one which is suitable for topical application to the skin and should include a solvent for the first polymer components of the film eg. that employed for a skin contacting layer. Physiologically acceptable solvents include aqueous solvents such as water itself, and organic solvents such as isopropanol, industrial methylated spirits, ethyl acetate, acetone, methyl ethyl ketone, dimethyl sulphoxide or mixtures thereof.

A preferred solvent, particularly for use with polyurethanes is isopropanol or an aqueous solution therof.

The liquid component may be formulated to provide a skin preparation liquid of the type normally used prior to surgical operation.

Suitably the filmic composition containing the second polymer, the liquid containing the first polymer or both may also contain a medicament such as an antibacterial agent.

Suitable antibacterial agents include chlorhexidine and salts thereof such as the acetate, gluconate or hydrochloride, iodophors such as polyvinyl pyrrolidone-iodine, silver salts such as silver sulphadiazine and polymeric biguanides for example those antibacterial agents known as Vantocil (Trade mark).

In those cases where the antibacterial agent is included in the liquid component of the invention it may be present in amounts ranging from 0.5 to 5% by weight. Preferably the liquid contains from 2.5 to 4.0% by weight of antibacterial agent. Alternatively if an antibacterial agent is to be included in the filmic component it may be present in amounts ranging from 1 to 10% by weight of the film. Preferably, about 5% by weight antibacterial agent is included in the film.

The dressings and filmic compositions of the present invention have applications for surgical incise drapes, I.V. dressings and wound dressings or the like. For use as a drape or I.V. dressing it may be convenient to apply the liquid component, for example by painting, or spraying the liquid in the general area or area where adherency is required, followed by application of the filmic component. The first polymer contained in the liquid composition will adhere readily to the filmic composition containing the second polymer even when the solvent for the first polymer has evaporated prior to bonding.

For wound care applications the liquid component may be applied around the edges of the wound site and thereafter eg. when the solvent has evaporated a sheet of filmic material applied over both the wound site and the area to which the liquid has been applied will the second polymer-containing filmic composition adhere; the dressing in the region over the wound site will remain unaffected. Thus the dressing in accordance with the invention can form effective window dressings.

Because the dressings of the invention are two component systems, and the final size of the dressing will depend only on the size of the area to which the liquid component is applied, it is not necessary to provide the filmic component in specific sizes. The filmic component can be provided in fewer sizes and cut down if necessary to the actual size and/or shape required. Furthermore since the film is not selfadherent it can be managed and handled easily.

Where it is necessary or desirable to ensure that all of the filmic component is adhered, the liquid component can be applied over a larger area than that required for the dressing.

When the solvent has dried off. The film of skin adhering first polymer formed from the liquid component will have a substantially non-adhesive surface and will only adhere and block to like polymers. Such a film therefore will not have a pressure sensitive adhesive surface which could be contaminated or lifted off by adherency to an operator's clothing for example gloves or a film carrier when applying a backing film of the second polymer to the skin adhering film. The adhesion of the first polymer film to the skin and the second polymer film should be sufficient to hold the dressing in place on the skin during healing or the time it is expected to operate However, it is preferred that the adhesion of first polymer film to the skin is less than that of the adhesion of this film to the second polymer film (backing film) to ensure that the formed dressing can be removed intact from the skin when required.

It may be preferred to coat the filmic composition with an antiblocking agent such as Gaisil, especially if the film is employed as a roll. The effect of the antiblocking agent is neutralised when the film contacts the liquid component.

The invention will now be illustrated by the following example.

### Example 1

A series of hydrophilic polyurethanes, P1 to P6, was prepared from 3 moles of polyethylene glycol (PEG600), 3 moles of polypropylene glycol (PPG600), from 3 to 13 moles of ethane diol (ED) and 4,4¹⁻dicyclohexyl methane di-isocyanate (Desmodur W) in an amount sufficient to maintain the overall NCO/OH ratio at 1:1. The polymers were prepared by adding the polyethylene glycol to polypropylene glycol, ethane diol and a dibutyltin dilaurate catalyst (T12) in an amount of 0.2% by weight of the total weight of the reactants to a reaction vessel. The vessel was placed into a fan assisted oven set at 60°C to melt the polyethylene glycol. When the polyethylene glycol had melted the mixture was well stirred and the 4,4'-dicyclohexylmethane di-isocyanate added with continued stirring. Stirring was further continued until the polymerisation mixture has changed from an opaque liquid to a clear one. At this point the temperature of the reaction mass was raised to 90°C and left for an hour to cure. The elastomer was cured for a further 24 hours at ambient temperature before being broken up and dissolved in 95:5 IPA/Water Mixture to provide a 25% solids solution.

The composition of the polymers was as shown in Table 1:

**Table 1**

| Polymer | ED Moles | PEG Moles | PPG Moles |
|---|---|---|---|
| P1 | 3 | 3 | 3 |
| P2 | 5 | 3 | 3 |
| P3 | 6 | 3 | 3 |
| P4 | 8 | 3 | 3 |
| P5 | 10 | 3 | 3 |
| P6 | 13 | 3 | 3 |
| P1 and P2 represent Comparative Examples. | | | |

A subjective assessment of the residual tackiness was made when samples of each of the polymers was cast using a 0.07mm spreading block onto a 0.15mm microporous embossed plasticised PVC film (Porvic). After the bulk of the solvent has evaporated the polymer coating was lightly touched with the finger. The results are shown in Table 2:

**Table 2**

| Polymer | ED(moles) | Assessment |
|---|---|---|
| P1 | 3 | tacky surface |
| P2 | 5 | tacky surface |
| P3 | 7 | slightly tacky surface |
| P4 | 9 | virtually no tack on surface |
| P5 | 11 | no tack on surface |
| P6 | 13 | no tack on surface. |

It will be apparent from the above results that diol/glycol ratios of greater than 1:1 are necessary to avoid pick-up of dirt on surplus uncontacted primer.

The 25% wt solution of polymers P1 to P6 were spread onto Porvic film supported on a card base and Estane film (0.035mm thick) was applied to test surfaces of the polymer 1, 5 and 30 minutes after the polymer had been spread, herein referred to as "contact time".

After contact with the Estane film, a 2kg roller was run over the contacted film and the samples left for 18 hours. At the end of this 2.54cm wide samples were cut out and subjected to a T-Peel Adhesion Test using an Instron Tensile Testing Machine. The cross-head speed was 300mm min⁻¹. The peel force was recorded on a chart also running at 300mm min⁻¹ . Full scale deflection was set to 1 or 2 kg f as required. The results are shown in Table 3.

**Table 3**

| Polymer | Adhesive Failure(mjm⁻²) at Contact Time | | |
|---|---|---|---|
| | 1 min | 5 min | 30 min |
| P1 | 125 | 120 | 115 |
| P2 | 162 | 80 | 105 |
| P3 | 60 | 45 | 55 |
| P4 | 75 | 70 | 45 |
| P5 | 65 | 30 | 0 |
| P6 | 55 | 0 | - |

The decrease in peel energy with increase in ethane diol content for contact times of 1 and 5 minutes is believed to be attributable to the residual solvent present in the polymer film. At higher ethane diol contents adhesive failure at corresponding lower peel energies for increasing ethane diol content is because of the increasing incompatibility between the hydrophilic polyurethane coating and Estane film.

### Example 2

A polymer P7 was produced by reacting PEG600 (3 moles), PPG600 (3 moles), ethane diol (8 moles) and Desmodur W, sufficient to maintain the NCO/OH ratio at 1:1, according to the method described in Example 1.

Five samples of a solution of polymer P7 in a mixture (95:5) of isopropanol and water were spread each at a different thickness on a supported Porvic film. After the lapse of 30 minutes to allow solvent to evaporate from the coating, Estane film was applied to the coating using the 2kg roller. The peel energy at which adhesive failure occured was determined using the Instron tester and the results are shown in Table 4:

**Table 4**

| Polymer | Solids Content of Solution %wt | Coating Thickness(mm) | Peel Energyjm⁻² |
|---|---|---|---|
| P7/1 | 25 | 0.006 | 0 |
| P7/2 | 25 | 0.019 | 50 |
| P7/3 | 25 | 0.031 | 90 |
| P7/4 | 25 | 0.038 | 87 |
| P7/5 | 8 | 0.06 | 100 |

Adjustments of the thickness affects the adhesion between the primer (first component) layer and the backing (second component) layer.

### Example 3

A 12.5% solution of polymer P5 in a 95:5 mixture of isopropanol and ethanol, was sprayed into the palm of the hand using a 1.4ml chamber pump action spray bottle. After 45 seconds, allowed for evaporation of isopropanol, a piece of estane film was placed on top and smoothed over. The film adhered well and had gentle peel for removal, comparible to the removal of a conventional adhesive coated Estane film (OpSite).

Adhesion failure occured at the primer/skin interface since peel of the film left little polymer P5 residue on the skin.

## Claims

1. A two component system for forming a dressing on the skin which comprises a first component comprising a solution of a film forming non-tacky skin adhering hydrophilic polyether polyurethane comprising either blocks derived from polyethylene glycol and polypropylene glycol and further comprising chain extenders such as aliphatic diols or aromatic diamines and a second component comprising a non-skin adhering film of a second polymer which will autohesively bond to a film of the first polymer characterised in that the chain extenders and glycols are present in a mole ratio of chain extender to glycol in the range of from 2:1 to 1:1.

2. A system as claimed in claim 1 wherein the second polymer is insoluble in the solution of the first polymer or the solvent therefor.

3. A system as claimed in claim 1 in which the solvent is an organic solvent.

4. A system as claimed in claim 3 in which the solvent is isopropanol.

5. A system as claimed in any of the preceeding claims wherein both polymers are polyurethanes.

6. A system as claimed in claim 5 in which the polyurethane is a polyether polyurethane.

7. A system as claimed in any one of the preceeding claims in which the film of second polymer has a thickness of 10 to 80µm.

8. A system as claimed in any one of claims 1 to 7 in which the first component polymer comprises a hydrophilic polyurethane which contains 5 to 50% of water when hydrated.

9. A system as claimed in claim 8 in which the polyurethane comprises residues of a chain extending aliphatic diol of up to 4 carbon atoms.

10. A dressing system as claimed in any of claims 1 to 9 in which either or both of the first and second components contain a medicament.

11. A dressing system as claimed in claim 10 in which the medicament is a topical antibacterial agent.

12. A system as claimed in any of claims 1 to 11 in which the dressing is an incise drape.

13. A system as claimed in any of claims 1 to 12 in which the second component film is in the form of a sheet or a continous roll.

14. A system as claimed in any of claims 1 to 13 in which the first liquid component is adapted to be applied to the skin directly, in the form of a spray or from a wipe.

15. A dressing comprising a non-tacky skin-adhering hydrophilic polyether polyurethane comprising ether blocks derived from polyethylene glycol and polypropylene glycol and further comprising chain extenders such as aliphatic diols or aromatic diamines autohesively bonded to a non-skin adhering film of a second polymer characterised in that the chain extenders and glycols are present in a mole ratio of chain extender to gylcol in the range from 2:1 to 1:1.

16. A dressing as claimed in claim 15 which is an incise drape.

17. A dressing as claimed in either of claims 15 or 16 which is moisture vapour transmitting and bacteria proof.

18. Use of a two component system as claimed in any one of claims 1 to 9 to form a dressing as claimed in any one of claims 10 to 17.

## Patentansprüche

1. Zweikomponentensystem zum Bilden eines Verbands auf der Haut, welches einen ersten Bestandteil umfaßt, der eine Lösung eines filmbildenden, nicht-klebrigen, hauthaftenden, hydrophilen Polyether-Polyurethans umfaßt, welches von Polyethylenglykol und Polypropylenglykol abgeleitete Etherblöcke enthält und weiter Kettenverlängerungsmittel, wie etwa aliphatische Diole oder aromatische Diamine, enthält und einen zweiten Bestandteil umfaßt, der einen nicht-hauthaftenden Film aus einem zweiten Polymer umfaßt, welcher selbstklebend an einen Film aus dem ersten Polymer bindet, dadurch gekennzeichnet, daß die Kettenverlängerungsmittel und Glykole in einem Molverhältnis Kettenverlängerungsmittel zu Glykol im Bereich von 2:1 bis 1:1 vorhanden sind.

2. System wie in Anspruch 1 beansprucht, in welchem das zweite Polymer in der Lösung des ersten Polymers oder dem Lösungsmittel dafür unlöslich ist.

3. System wie in Anspruch 1 beansprucht, in welchem das Lösungsmittel ein organisches Lösungsmittel ist.

4. System wie in Anspruch 3 beansprucht, in welchem das Lösungsmittel Isopropanol ist.

5. System wie in einem der vorangehenden Ansprüche beansprucht, in welchem beide Polymere Polyurethane sind.

6. System wie in Anspruch 5 beansprucht, in welchem das Polyurethan ein Polyether-Polyurethan ist.

7. System wie in einem der vorangehenden Ansprüche beansprucht, in welchem der Film aus dem zweiten Polymer eine Dicke von 10 bis 80 µm besitzt.

8. System wie in einem der Ansprüche 1 bis 7 beansprucht, in welchem der erste Polymerbestandeil ein hydrophiles Polyurethan umfaßt, welches 5 bis 50% Wasser enthält, wenn es gewässert wird.

9. System wie in Anspruch 8 beansprucht, in welchem das Polyurethan Reste eines kettenverlängernden aliphatischen Diols mit bis zu 4 Kohlenstoffatomen umfaßt.

10. Verbandsystem wie in einem der Ansprüche 1 bis 9 beansprucht, in welchem entweder einer oder beide ersten und zweiten Bestandteile ein Arzneimittel enthalten.

11. Verbandsystem wie in Anspruch 10 beansprucht, in welchem das Arzneimittel ein topisches antibakterielles Mittel ist.

12. System wie in einem der Ansprüche 1 bis 11 beansprucht, in welchem der Verband ein chirurgisches Abdecktuch ist.

13. System wie in einem der Ansprüche 1 bis 12 beansprucht, in welchem der zweite Filmbestandteil in Form eines Bogens oder einer ununterbrochenen Rolle vorliegt.

14. System wie in einem der Ansprüche 1 bis 13 beansprucht, in welchem der erste flüssige Bestandteil daran angepaßt ist, in der Form eines Sprays oder von einem Wischtuch direkt auf die Haut aufgebracht zu werden.

15. Verband, welcher ein nicht-klebriges, hauthaftendens, hydrophiles Polyether-Polyurethan umfaßt, das von Polyethylenglykol und Polypropylenglykol abgeleitete Etherblöcke enthält und weiter Kettenverlängerungsmittel, wie etwa aliphatische Diole oder aromatische Diamine, enthält und selbsthaftend an einen nicht-hauthaftenden Film aus einem zweiten Polymers gebunden ist, dadurch gekennzeichnet, daß die Kettenverlängerungsmittel und Glykole in einem Molverhältnis Kettenverlängerungsmittel zu Glykol im Bereich von 2:1 bis 1:1 vorhanden sind.

16. Verband wie in Anspruch 15 beansprucht, welcher ein chirurgisches Abdecktuch ist.

17. Verband wie entweder in Anspruch 15 oder 16 beansprucht, welcher feuchtigkeitsdampfdurchlässig und bakteriendicht ist.

18. Verwendung eines in einem der Ansprüche 1 bis 9 beanspruchten Zweikomponentensystems zum Bilden eines in einem der Ansprüche 10 bis 17 beanspruchten Verbands.

## Revendications

1. Système à deux composants pour former un pansement sur la peau, qui comprend un premier composant comportant une solution d'un polyéther polyuréthane hydrophile adhérant à la peau, non poisseux et filmogène comprenant des blocs éthers dérivés du polyéthylèneglycol ou du polypropylène glycol et comprenant en outre des éléments d'extension de chaîne tels que des diols aliphatiques ou des diamines aromatiques, et un deuxième composant comportant un film non adhérant à la peau d'un deuxième polymère qui se lie par auto-adhérence à un film du premier polymère, caractérisé en ce que les éléments d'extension de chaîne et les glycols sont présents dans un rapport molaire de l'élément d'extension de chaîne au glycol compris entre 2:1 et 1:1.

2. Système suivant la revendication 1, dans lequel le deuxième polymère est insoluble dans la solution du premier polymère ou dans le solvant pour cette solution.

3. Système suivant la revendication 1, dans lequel le solvant est un solvant organique.

4. Système suivant la revendication 3, dans lequel le solvant est l'isopropanol.

5. Système suivant une quelconque des revendications précédentes, dans lequel les deux polymères sont des polyuréthanes.

6. Système suivant la revendication 5, dans lequel le polyuréthane est un polyéther polyuréthane.

7. Système suivant une quelconque des revendications précédentes, dans lequel le film du deuxième polymère a une épaisseur de 10 à 80µm.

8. Système suivant une quelconque des revendications 1 à 7, dans lequel le polymère du premier composant comprend un polyuréthane hydrophile qui contient 5 à 50% d'eau lorsqu'il est hydraté.

9. Système suivant la revendication 8, dans lequel le polyuréthane comprend des résidus d'un diol aliphatique d'extension de chaîne ayant jusqu'à 4 atomes de carbone.

10. Système de pansement suivant une quelconque des revendications 1 à 9, dans lequel l'un ou l'autre des premier et deuxième composants ou les deux contiennent un médicament.

11. Système de pansement suivant la revendication 101 dans lequel le médicament est un agent antibactérien topique.

12. Système suivant une quelconque des revendications 1 à 11, dans lequel le pansement est un drap d'incision.

13. Système suivant une quelconque des revendications 1 à 12, dans lequel le film constituant le deuxième composant est sous la forme d'une feuille ou d'une bobine continue.

14. Système suivant une quelconque des revendications 1 à 13, dans lequel le premier composant liquide est prévu pour être appliqué à la peau directement, sous la forme d'un jet pulvérisé ou à l'aide d'un tampon.

15. Pansement comprenant un polyéther polyuréthane hydrophile adhérant à la peau et non poisseux, comportant des blocs éther dérivés du polyéthylène glycol et du polypropylène glycol et comportant en outre des éléments d'extension de chaîne tels que des diols aliphatiques ou diamines aromatiques, lié par auto-adhérence à un film non adhérant à la peau d'un deuxième polymère, caractérisé en ce que les éléments d'extension de chaîne et les glycols sont présents dans un rapport molaire de l'élément d'extension de chaîne au glycol compris entre 2:1 et 1:1.

16. Pansement suivant la revendication 15, qui est un drap d'incision.

17. Pansement suivant une quelconque des revendications 15 ou 16, qui transmet la vapeur d'eau et arrête les bactéries.

18. Utilisation d'un système à deux composants suivant une quelconque des revendications 1 à 9, pour former un pansement suivant une quelconque des revendications 10 à 17.
